(19) Europäisches Patentamt
European Patent Office
Office européen des brevets

(11) **EP 2 305 098 A1**

(12) **EUROPEAN PATENT APPLICATION**
published in accordance with Art. 153(4) EPC

(43) Date of publication:
**06.04.2011 Bulletin 2011/14**

(51) Int Cl.:
***A61B 1/12*** *(2006.01)*

(21) Application number: **09800302.3**

(86) International application number:
**PCT/JP2009/061717**

(22) Date of filing: **26.06.2009**

(87) International publication number:
**WO 2010/010787 (28.01.2010 Gazette 2010/04)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **24.07.2008 JP 2008191225**

(71) Applicant: **Olympus Medical Systems Corp.**
**Tokyo 151-0072 (JP)**

(72) Inventors:
• **TOMITA Masahiko**
**Tokyo 151-0072 (JP)**
• **NOGUCHI, Toshiaki**
**Tokyo 151-0072 (JP)**

• **ONISHI, Hideto**
**Tokyo 151-0072 (JP)**
• **HASEGAWA, Hitoshi**
**Tokyo 151-0072 (JP)**
• **SEWAKE, Ryuta**
**Tokyo 151-0072 (JP)**
• **NAKANISHI, Nobuyki**
**Tokyo 151-0072 (JP)**
• **NOZAKI, Satoshi**
**Tokyo 151-0072 (JP)**

(74) Representative: **Schicker, Silvia**
**Wuesthoff & Wuesthoff**
**Patent- und Rechtsanwälte**
**Schweigerstrasse 2**
**81541 München (DE)**

(54) **DEVICE FOR AUTOMATICALLY CLEANING AND STERILIZING ENDOSCOPE**

(57)     A control section 201 reads data such as a conduit diameter and a conduit length of an endoscope 100 and a cleaning profile which are separately recorded in a storage section 202 based on type information of the endoscope 100 registered in the storage section 202. A cleaning degree suited to the endoscope 100 is estimated by calculation based on a pressure value and a flow rate value which are measured, the diameter and the length of the conduit to be cleaned, and the cleaning profile.

**FIG.1**

EP 2 305 098 A1

**Description**

Technical Field

The present invention relates to an endoscope automatic cleaning/disinfecting apparatus which cleans and disinfects an endoscope after use.

Background Art

In recent years, endoscopes have been widely used in the medical field and the industrial field. An endoscope used in the medical field enables observation of the organs in a body by insertion of an elongated insertion portion into the body, and enables various kinds of treatments with use of the treatment instruments inserted in an insertion channel for the treatment instrument according to necessity.

In particular, the endoscopes in the medical field are used by being inserted into bodies for the purpose of inspection and medical treatment, and therefore, the endoscopes after use need to be cleaned and disinfected. When the endoscopes after use are cleaned and disinfected, endoscope cleaning/disinfecting apparatuses are sometimes used. In this case, an endoscope is set in a cleaning tank of an endoscope cleaning/disinfecting apparatus, and is cleaned, disinfected, rinsed and drained. An endoscope internally has a plurality of internal conduits such as an air supply/water supply channel and a forceps channel. A cleaning liquid and an antiseptic solution need to be sufficiently passed inside the internal conduits, and the internal conduits also need to be cleaned and disinfected.

Conventionally, for example, in the endoscope cleaning/disinfecting apparatus of Japanese Patent Application Laid-Open Publication No. 2006-230709 or the like, treatment solutions such as water, a cleaning liquid and an antiseptic solution, or air is fed to endoscope conduits by a pump, compressor and the like incorporated in the cleaning machine. In order to clean the endoscope conduits, a treatment solution, air or a mixture fluid of them is passed into the endoscope conduits, and thereby, the filth presents inside the endoscope is removed. In particular, when the filth adhering to the inner walls of the endoscope conduits is to be removed, the filth is removed by application of a shearing force with a certain magnitude or more to the inner wall.

However, there is the problem of being incapable of evaluating the cleaning performance for the inner wall of an endoscope conduit in the conventional endoscope cleaning/disinfecting apparatus.

The present invention is made in the above described circumferences, and has an object to provide an endoscope automatic cleaning/disinfecting apparatus capable of indirectly evaluating cleaning power for an endoscope inner wall by using a measurement result of a fluid state in an endoscope conduit.

Disclosure of Invention

Means for Solving the Problem

An endoscope automatic cleaning/disinfecting apparatus of the present invention is, in an endoscope automatic cleaning/disinfecting apparatus which cleans and disinfects an endoscope, configured by including fluid supply means for supplying a fluid to an endoscope conduit disposed inside the endoscope, a fluid supply conduit which is connected to the fluid supply means, and supplies the fluid to the endoscope conduit, a connection connector which is provided at a distal end at a side of the endoscope, of the fluid supply conduit, and is for connecting the fluid supply conduit to the endoscope, fluid measuring means which measures a state of the fluid in the connection connector the fluid supply conduit, and control means which calculates a cleaning evaluation value of the endoscope conduit based on a measurement value obtained by the fluid measuring means, and controls the fluid supply means in accordance with a result of the calculation.

Brief Description of the Drawings

Fig. 1 is a general view showing an endoscope automatic cleaning/disinfecting apparatus according to embodiment 1 of the present invention;
Fig. 2 is a configuration diagram showing a configuration of the endoscope automatic cleaning/disinfecting apparatus of Fig. 1;
Fig. 3 is a flowchart explaining an operation of a control section of Fig. 2;
Fig. 4 is a flowchart showing a control flow according to embodiment 2 of the present invention;
Fig. 5 is a diagram showing one example of data written in a storage section in advance in embodiment 3 of the present invention; and
Fig. 6 is a flowchart showing a control flow according to embodiment 3 of the present invention.

Best Mode for Carrying Out the Invention

Hereinafter, embodiments of the present invention will be described with reference to the drawings.

(Embodiment 1)

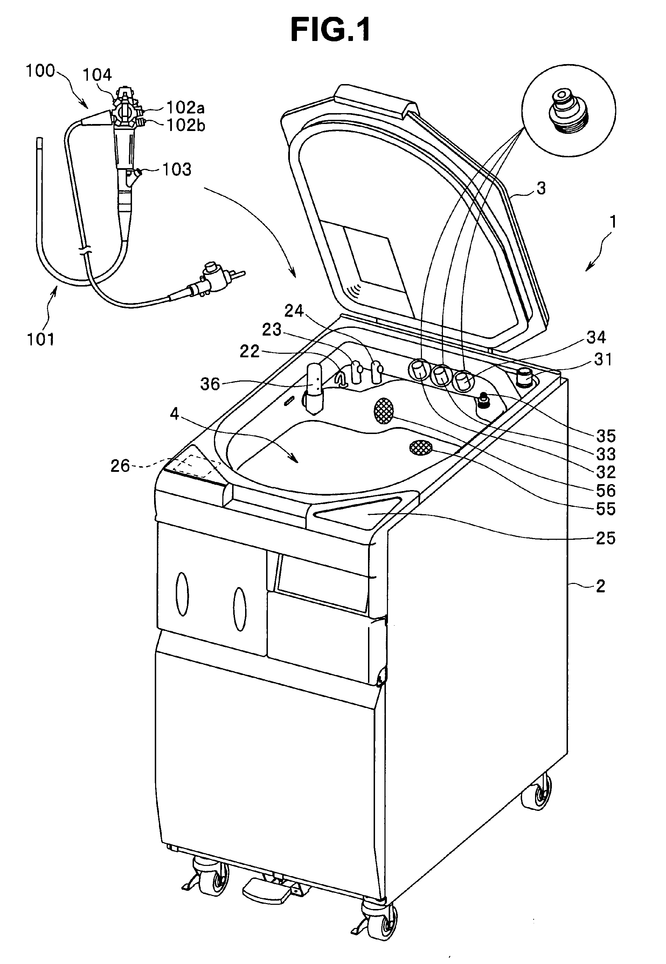

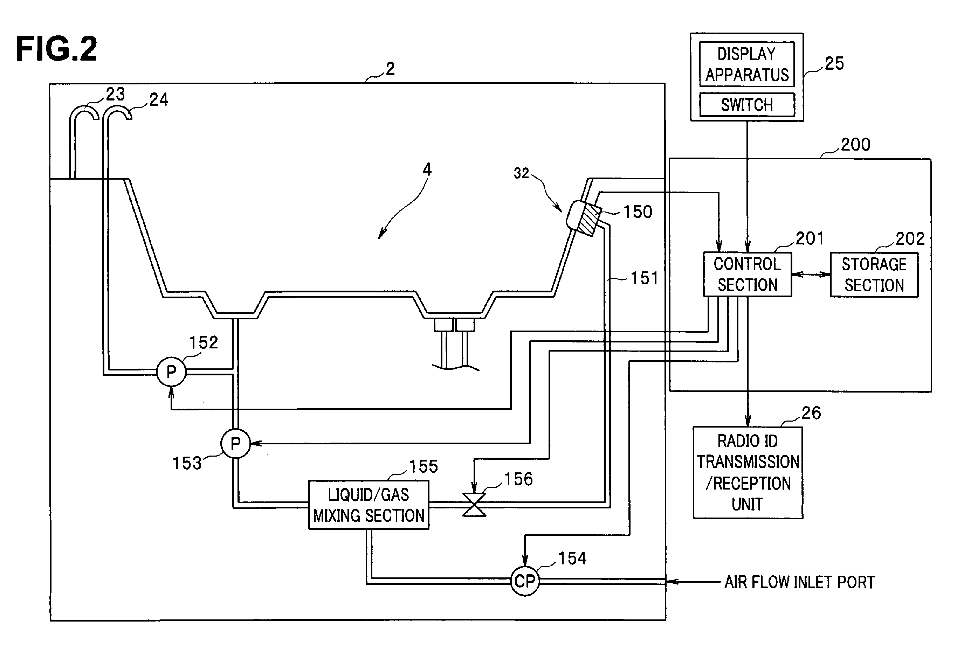

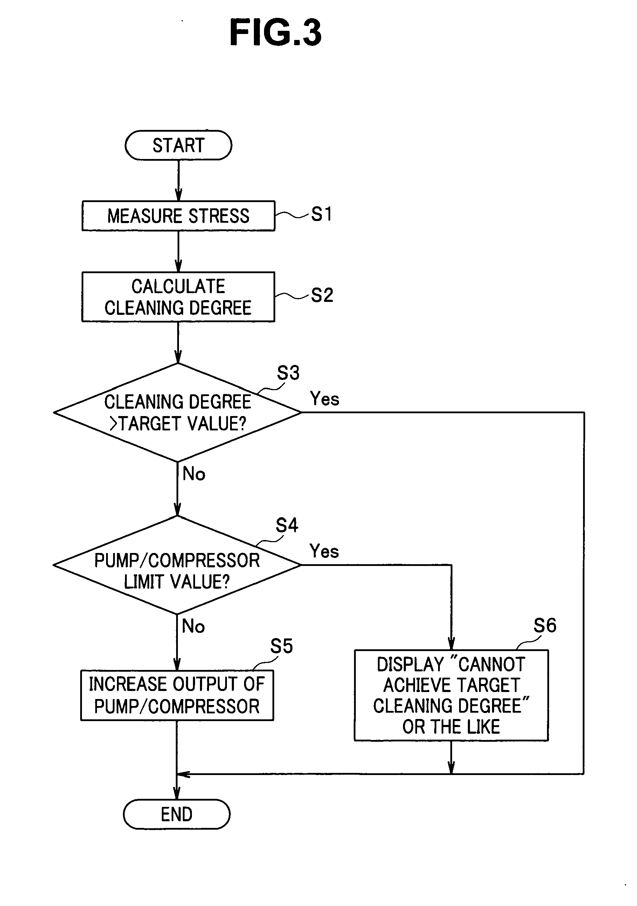

Figs. 1 to 3 relate to embodiment 1 of the present invention. Fig. 1 is a general view showing an endoscope automatic cleaning/disinfecting apparatus. Fig. 2 is a configuration diagram showing an internal configuration of the endoscope automatic cleaning/disinfecting apparatus of Fig. 1. Fig. 3 is a flowchart explaining an operation of a control section of Fig. 2.

As shown in Fig. 1, an endoscope automatic cleaning/disinfecting apparatus 1 of the present embodiment is an apparatus for cleaning and disinfecting an endoscope 100 after use, and a main part is configured by an apparatus main body 2, and a top cover 3 which is a lid body connected to an upper portion of the apparatus main body 2 via, for example, a hinge not illustrated to be openable and closable.

A cleaning/disinfecting tank 4 for housing the endoscope 100 is provided at a substantially central portion of a top surface of the apparatus main body 2. The endoscope 100 is set inside the cleaning/disinfecting tank 4, the top cover 3 is closed, and a predetermined operation is performed, whereby cleaning and disinfection of the endoscope 100 is automatically performed by using a liquid such as a cleaning liquid which is a fluid.

In the cleaning/disinfecting tank 4, a detergent nozzle 22 for supplying a detergent to clean and disinfect an outer surface of the endoscope 100, a disinfection nozzle 23 for supplying an antiseptic solution, and a supply water circulation nozzle 24 for supplying a circulating liquid are provided. Further, in the cleaning/disinfecting tank 4, a circulation port 56 for recovering the liquid in the cleaning/disinfecting tank 4 to circulate the liquid, and a drain port 55 for draining the liquid in the cleaning/disinfecting tank 4 are also provided.

Further, as shown in Fig. 1, an operation panel 25 on which setting switches such as a cleaning/disinfecting operation start switch, and a cleaning/disinfecting mode selection switch of the apparatus main body 2 are arranged and a display apparatus is included, is provided at a portion closer to the right of a front surface side, which, for example, an operator approaches, on the top surface of the apparatus main body 2. A radio ID transmission/reception unit 26 which receives an identification signal from an identification signal transmitter such as an ID tag provided in the endoscope 100 is incorporated in a portion closer to the left of the front surface side of the top surface of the apparatus main body 2. The identification signal is, for example, a code signal or the like showing the kind of the endoscope 100.

Further, a water supply hose connection port 31 to which a hose connected to a water tap is connected, and which is for supplying service water to the apparatus main body 2 is arranged at a side which is opposite from the front surface which the operator approaches, and is on the top surface of the apparatus main body 2. A filter for filtering service water may be arranged in the water supply hose connection port 31.

Further, in the cleaning/disinfecting tank 4, a suction conduit connector 32, an air-supply and water-supply conduit connector 33, a forceps raising pipe connector 34, and a water leakage detection port connector 35 that are connecting means which are respectively connected to opening portions of respective channels of the endoscope 100 via connecting tubes are arranged to clean and disinfect the inside of the respective channels which are endoscope conduits inside the endoscope 100. Further, the connectors 32, 33 and 34 each have the mechanism which communicates with a connection tube when the connection tube is connected thereto, and is closed when the connection tube is not connected thereto. Further, a water level sensor 36 for detecting a water level of the liquid inside the cleaning/disinfection tank 4 is provided inside the cleaning/disinfecting tank 4.

In general, the endoscope 100 not only picks up an image of the surface of a subject from a distal end of an insertion section 101, but also has a plurality of various channels which are internal endoscope conduits for performing air supply and water supply, performing suction, and inserting a treatment instrument such as forceps therethrough, at a distal end portion of the insertion section 101 of the endoscope 100 depending on the kind of the endoscope 100. In addition, the endoscope 100 has a channel or the like which is an internal conduit for raising forceps to change the direction to protrude in the distal end of the insertion section 101 in some cases.

Accordingly, when the endoscope 100 is set in the cleaning/disinfecting tank 4 to clean and disinfect the inside of each of the channels of the endoscope 100, one end of each of the connection tubes is connected to the opening portion of each of the channels of the endoscope 100, and the other end is connected to connection means such as the suction conduit connector 32, the air-supply and water-supply conduit connector 33, and the forceps raising pipe connector 34 inside the cleaning/disinfecting tank 4. As a concrete example, one end of one connection tube (suction channel connecting tube) is connected to a forceps port 103 of the endoscope 100, and the other end of the connection tube is connected to the suction conduit connector 32 in the cleaning/disinfecting tank 4. One end of another connection tube (air-supply and water-supply channel connecting tube) is connected to air-supply and water-supply cylinders 102a and 102b of the air-supply and water-supply channel of the endoscope 100, and the other end of the connection tube is connected to the air-supply and water-supply conduit connector 33 in the cleaning/disinfecting tank 4. One end of another connection tube (forceps raising channel connecting tube) is connected to a forceps raising port 104 of the forceps raising channel of the endoscope 100, and the other end of the connection tube is connected to the forceps raising pipe connector 34 in the cleaning/disinfecting tank 4. One end of still another connection tube (water leakage detecting connecting tube) is connected to a water leakage detection port (not illustrated) of the endoscope 100, and the other end of the connection tube is connected to the water leakage detection port connector 35 in the cleaning/disinfecting tank 4.

In order to enable cleaning and disinfection of a plurality of endoscopes 100 in one endoscope cleaning/disinfecting apparatus 1, a plurality of connectors such as the suction conduit connectors 32 connected to the internal channels may be provided respectively.

Next, a configuration of the endoscope automatic cleaning/disinfecting apparatus 1 of the present embodiment will be described with use of Fig. 2 with the suction conduit connector 32 as an example. The air-supply and water-supply conduit connector 33 and the forceps raising pipe connector 34 are similarly configured, and the explanation relating to the air-supply and water-supply conduit connector 33 and the forceps raising pipe connector 34 will be omitted. Further, hereinafter, the suction conduit connector 32 is described as the conduit connector 32.

As shown in Fig. 2, the endoscope automatic cleaning/disinfecting apparatus 1 of the present embodiment is configured by including the apparatus main body 2 and a control unit 200. In the apparatus main body 2, a treatment solution accumulating in the cleaning/disinfecting tank 4 is sucked by a pump 153, and is fed to the endoscope conduit via the fluid supply conduit 151 and the conduit connector 32, while the treatment solution accumulating in the cleaning/disinfecting tank 4 is sucked by a pump 152, and is circulated into the cleaning/disinfecting tank 4 through the supply water circulation nozzle 24.

Instead of the circulation type as in Fig. 2, a tank storing a treatment solution is additionally disposed, and the treatment solution may be directly fed out from the tank to the endoscope conduit.

Meanwhile, air is fed out to the endoscope conduit via the fluid supply conduit 151 and the conduit connector 32 by a compressor (or an air pump or the like) 154. Especially when the compressor is used, air is considered to be fed out by being compressed.

The treatment solution and air pass through the fluid supply conduit 151 as described above, and this is realized by a liquid/gas mixing section 155 and an electromagnetic valve 156 as mixing means which enables a liquid and a gas to merge and flow in the same direction in the fluid supply conduit, and in some cases, a liquid and a gas can be fed out at the same time.

The conduit connector 32 is a terminal of a cleaning machine that is directly connected to the endoscope 100 which is disposed in the cleaning tank to be subjected to cleaning and disinfecting treatment, or is connected to a tube (not illustrated) with one end connected to the endoscope 100 and the other end connected to the endoscope automatic cleaning/disinfecting apparatus 1, and supplies the treatment solution and air to the outside from the inside of the endoscope automatic cleaning/disinfecting apparatus 1.

In the present embodiment, a measuring apparatus 150 as measuring means which measures the pressure of a fluid passing inside the fluid supply conduit 151, a stress which the fluid exerts on the measuring means, a shearing stress which is exerted on a wall surface of the fluid supply conduit 151 or a wall surface of an endoscope conduit or the like is disposed at a side of the fluid supply conduit 151, of the conduit connector 32.

The filth adhering to the conduit wall surface of the endoscope 100 is removed by the action of the fluid flowing in the endoscope conduit, and this is removed when the fluid applies a shearing force which is equal to or more than the adhering force of the filth adhering to the conduit wall surface. For example, when the fluid is in a weak flow, the filth is hardly removed, whereas when the fluid is in a strong flow, the filth is easily removed, and this is because the shearing stresses applied to the conduit wall surface by the fluid differ from each other.

A shearing stress can be obtained based on the inner diameter of a conduit, a conduit length, pressure losses at both ends of the conduit and the like. In the configuration of the present embodiment, the conduit to be cleaned is an endoscope conduit, one end of the endoscope conduit is in an opened space of the cleaning tank, and the pressure of that portion is equal to an atmospheric pressure. The other end is connected to the conduit connector 32 of the endoscope automatic cleaning/disinfecting apparatus 1. Accordingly, if the measuring apparatus 150 (for example, a pressure sensor) which measures the pressure in the fluid supply conduit beside the conduit connector 32, or the pressure received from the fluid itself which flows in the fluid supply conduit is available, the pressure loss can be calculated, and the shearing stress can be obtained.

Accordingly, by measuring the pressure beside the conduit connector 32, the pressure loss can be calculated. Further, based on the shearing stress obtained from the pressure loss, and a certain fixed formula, a cleaning evaluation value is calculated, and the cleaning degree of the endoscope conduit can be estimated.

Further, by the measuring apparatus 150 (for example, a pressure sensor), the shearing stress which is discontinuously to and instantaneously applied to the wall can be measured. If the fluid stably flows, and therefore, the shearing stress becomes substantially average, the value of the measuring apparatus 150 (for example, the pressure sensor) is outputted as the value with less variation. However, in the case of the cleaning for which an instantaneously large shearing stress which occurs in a pulse form is important, instead of an average shearing stress, by intentionally causing an unstable flow to the fluid, the cleaning degree can be estimated by this method.

Especially in the case of a fluid in which a small number of small liquid droplets are contained in a high-speed gas flow, a large shearing stress occurs when a liquid with a large mass collides with the wall surface, and the measuring apparatus 150 (for example, the pressure sensor) measures a very large pressure at the instant. Accordingly, the cleaning evaluation value is calculated from the pressure value which occurs at this instant instead of pressure loss, and the cleaning degree can be estimated. In this case, in order to evaluate the cleaning degree in the endoscope conduit more precisely, the pressure is desirably measured in the vicinity of the conduit connector 32 by locating the measuring apparatus 150 (for example, the pressure sensor) as close to the endoscope 100 as possible. Further, the measuring apparatus 150 may be disposed so that the sensor portion is included in the fluid supply conduit, or may be disposed so that the sensor portion is in contact with the outer wall of the fluid supply conduit, and the disposition of the measuring apparatus 150 can be arbitrarily selected according to the measurement method.

The endoscope 100 generally has a different inner diameter and length according to the use purpose, and the endoscope automatic cleaning/disinfecting apparatus 1 needs to be adapted to various endoscopes which are assumed.

Accordingly, the control unit 200 of the endoscope automatic cleaning/disinfecting apparatus 1 of the present embodiment is configured by including a control section 201 and a storage section 202. The control section 201 specifies the type of the endoscope 100 to be cleaned and disinfected by automatically reading the information manually inputted from the operation panel 25 and the information of an RF-ID tag included in the endoscope through the radio ID transmission/reception unit 26. Further, the storage section 202 converts/corrects the cleaning evaluation value according to the type of the endoscope to be cleaned and disinfected which is compared with the value measured by the measuring apparatus 150 (for example, the pressure sensor), and stores the cleaning evaluation value.

Further, if the velocity of the fluid flowing in the fluid supply conduit is measured as well as the pressure, the shearing stress can be similarly obtained, and therefore, a flow rate sensor can be used as the measuring apparatus 150. In the fluid supply conduit 151, a fluid, a gas, and a two-phase fluid with a fluid and a gas mixed under some circumstances flow therein, and therefore, a flow rate sensor which can measure these fluids is desirable.

As shown in Fig. 3, the control section 201 of the control unit 200 acquires the measurement value of the pressure (or flow rate or the like) of the fluid supply conduit 151 from the measuring apparatus 150 in step S1. In step 2, the control section 201 calculates and converts a cleaning degree value from which the cleaning degree can be measured such as a shearing force, based on the measurement value measured by the measuring apparatus 150.

In the present embodiment, the control section 201 registers the type information of the endoscope 100 to be cleaned and disinfected in the storage section 202 by reading manual input from the operation panel 25 or the RF-ID of the endoscope 100 through the radio ID transmission/reception unit 26. The control section 201 reads the data of the conduit diameter, the conduit length and the like of the endoscope 100, and a cleaning profile configured by the information of the pressure or the flow rate of the supply fluid to be supplied to the endoscope conduit, or the mixture ratio of the supply fluid, the supply timing of the supply fluid, or the like which are separately recorded in the storage section 202 based on type information of the endoscope 100 registered in the storage section 202. Based on the measured pressure value and flow rate value, the diameter and the length of the conduit to be cleaned, and the cleaning profile, the cleaning degree suited to the endoscope 100 can be estimated by calculation. Further, by changing the operation of the pump 153, the compressor 154 and the liquid/gas mixing section 155, and the electromagnetic valve 156 for each kind of endoscope, the effective cleaning power can be applied to many kinds of endoscopes in accordance with the kinds.

The control section 201 determines whether or not the cleaning degree is lower than a fixed target value in step S3. When the cleaning degree is lower than the fixed target value, the control section 201 increases the cleaning degree by controlling the pump 153, the compressor 154 and the liquid/gas mixing section 155, and the electromagnetic valve 156. Meanwhile, the control section 201 finishes the processing when the cleaning degree reaches the fixed target value.

Subsequently, the control section 201 determines whether or not the pump 153 and the compressor 154 reach the upper limit in terms of performance in step S4. When the pump 153 and the compressor 154 do not reach the upper limit in terms of performance, the control section 201 increases the output of the pump 153 and the compressor 154 to finish the processing in step S5. Meanwhile, when the control section 201 determines that the pump 153 and the compressor 154 reach the upper limit in terms of performance, the control section 201 displays the warning to the effect that the target cleaning degree cannot be achieved (for example, "cannot achieve the target cleaning degree" or the like) on the display apparatus of the operation panel 25 in step S6 to inform an user of this to finish the processing.

Further, when the pressure in a pulse form is measured in the description of step S6, if the pulse is not present for a certain fixed time, the treatment solution such as a cleaning liquid necessary for cleaning is likely to run short, and by displaying (announcing) that effect on the display apparatus of the operation panel 25, the user can be informed of the shortage.

As above, in the present embodiment, the cleaning degree in the endoscope conduit can be estimated by the measuring apparatus 150 installed in the vicinity of the conduit connector. Further, the information can be recorded as a cleaning history.

Further, when the cleaning degree obtained based on the measurement value of the measuring apparatus 150 of the fluid supply conduit 151 is low, the output can be increased for the pump 153 and the compressor 154, and the target cleaning power can be pursued.

Further, when the performance of the pump 153, the compressor 154 and the like cannot be increased any more, or when the measurement of the performance cannot be obtained for a fixed time, the user can confirm presence or absence of the cleaning liquid, and can know the failure or the like of the apparatus by letting the user know that the target cleaning degree cannot be achieved, and an inadequacy in cleaning due to a malfunction of the endoscope automatic cleaning/disinfecting apparatus 1 can be avoided.

(Example 2)

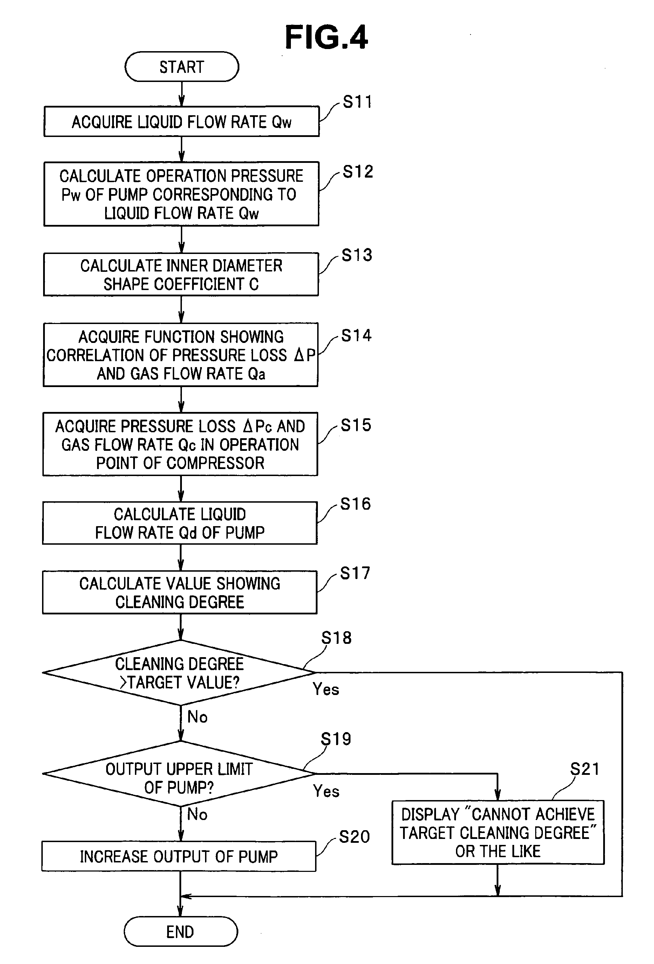

Fig. 4 relates to embodiment 2 of the present invention. In the present embodiment, the case will be described, in which the measuring apparatus 150 is configured as a liquid flowmeter which measures the flow rate of a liquid. In the following description, the detailed description of the components having the configurations similar to those in embodiment 1 will be omitted.

In the storage section 202 of the present embodiment, the function showing the correlation between the operation pressure of the pump 153 and the liquid flow rate as operation characteristic data of the pump 153 which is obtained based on a rated value, an actual measurement value or the like is written in advance. Further, in the storage section 202 of the present embodiment, the function showing the correlation between the operation pressure of the compressor 154 and the gas flow rate as operation characteristic data of the compressor 154 which is obtained based on a rated value, an actual measurement value or the like is written in advance. Further, in the storage section 202 of the present embodiment, the optimal mixture ratio of the gas and the liquid in the supply fluid which is supplied to the endoscope is written in advance.

Meanwhile, the control section 201 of the control unit 200 acquires the measurement value of the flow rate of the liquid flowing in the fluid supply conduit 151 from the measuring apparatus 150 in step S11 of Fig. 4. Subsequently, the control section 201 regards the measurement value acquired from the measuring apparatus 150 as a flow rate Qw of the liquid flowing in the channel of the endoscope 100, and reads the operation characteristic data of the pump 153 from the storage section 202, in step S11 of Fig. 4.

Next, in step S12 of Fig. 4, the control section 201 acquires an operation pressure Pw of the pump 153 corresponding to the aforesaid liquid flow rate Qw by checking the flow rate Qw of the liquid flowing in the channel of the endoscope 100 and the operation characteristic data of the pump 153.

Thereafter, in step S13 of Fig. 4, the control section 201 substitutes the respective values of the flow rate Qw of the liquid flowing in the channel of the endoscope 100, and the operation pressure Pw of the pump 153 corresponding to the aforesaid liquid flow rate Qw into the following expression (1), and thereby, calculates an inner diameter shape coefficient C of the channel of the endoscope 100.

$$C = Pw \big/ \lambda w \cdot \rho w \cdot Qw^2 \quad \cdots \quad (1)$$

$\lambda w$ of the above described expression (1) represents a frictional coefficient in conduit at the time of the liquid flowing in the channel of the endoscope 100. In concrete, the frictional coefficient $\lambda w$ in the conduit is a frictional coefficient at the time of the cleaning liquid flowing in the channel of the endoscope 100, and is a calculation value which is obtained by performing calculation or the like with consideration given to, for example, the actual measurement value shown by the measuring apparatus which is obtained when the cleaning liquid is actually passed into the channel of the endoscope 100, the material, the surface roughness, the conduit diameter and the like of the channel of the endoscope 100. The material and the surface roughness are specified to be several patterns according to the kind of an endoscope. The conduit diameter is finitely set in 0.1 mm increments between $\phi$0.5 mm to $\phi$6.0 mm, for example. Therefore, the table of $\lambda w$ corresponding to the conduit diameter is stored in the storage section 202, and is read by the control section 201 before the calculation of the above described expression (1) is performed, and thereby, the calculation step of $\lambda w$ can be omitted.

$\rho w$ of the above described expression (1) represents a liquid density. In concrete, the liquid density $\rho w$ is, for example, the density of the cleaning liquid which is used at the time of cleaning the endoscope 100, and is a fixed value corresponding to the kind of the detergent and the water quality in the aforesaid cleaning liquid. The liquid density $\rho w$ is written in the storage section 202 in advance, and is read by the control section 201 before calculation of the above described expression (1) is performed.

Subsequently, in step S14 of Fig. 4, the control section 201 substitutes the value of the inner diameter shape coefficient C into the following expression (2), and thereby, acquires the function (quadric function) showing the correlation between a pressure loss ΔP and a gas flow rate Qa.

$$\Delta P = \lambda a \cdot \rho a \cdot C \cdot Qa^2 \quad \cdot \cdot \cdot \quad (2)$$

λa of the above described expression (2) represents the frictional coefficient in the conduit at the time of a gas flowing in the channel of the endoscope 100. In concrete, the frictional coefficient λa in the conduit is a frictional coefficient at the time of a gas flowing in the channel of the endoscope 100, and is a calculation value which is obtained by performing calculation or the like with consideration given to, for example, an actual measurement value shown by the measurement value obtained when air is actually passed into the channel of the endoscope 100, and the material and the surface roughness of the channel of the endoscope 100. The frictional coefficient λa in the conduit can be stored in the storage section 202 by the similar method to the aforesaid λw, and is read by the control section 201 before calculation of the above described expression (2) is performed, whereby the calculation step of λa can be omitted.

ρa of the above described expression (2) represents a gas density. In concrete, the gas density ρa is, for example, the density of the gas which is used at the time of cleaning the endoscope 100, and is a fixed value obtained based on the density of the aforesaid gas, for example. The liquid density ρa is written in the storage section 202 in advance, and is read by the control section 201 before calculation of the above described expression (2) is performed.

In step S15 of Fig. 4, the control section 201 acquires a pressure loss ΔPc and a gas flow rate Qc in the operation point of the compressor 154 by checking the function obtained by substituting the value of the inner diameter shape coefficient C into the above described expression (2) and the operation characteristic data of the compressor 154. In concrete, the control section 201 acquires the pressure loss ΔPc and the gas flow rate Qc in the operation point of the compressor 154 by calculating the intersection of the quadric function obtained by substituting the value of the inner diameter shape coefficient C into the above described expression (2) and the function corresponding to the operation characteristic data of the compressor 154.

The control section 201 reads an optimal mixture ratio of the gas and the liquid in the supply fluid supplied to the endoscope 100 from the storage section 202, and thereafter, calculates a liquid flow rate Qd of the pump 153 by dividing the gas flow rate Qc by the aforesaid mixture ratio in step S16 of Fig. 4.

Thereafter, in step S17 of Fig. 4, the control section 201 calculates the value indicating the cleaning degree in the channel of the endoscope 100 by obtaining the ratio of the liquid flow rate Qw and the liquid flow rate Qd, for example.

In step S18 of Fig. 4, the control section 201 determines whether or not the value indicating the cleaning degree in the channel of the endoscope 100 exceeds a fixed target value. When the value indicating the cleaning degree in the channel of the endoscope 100 is the fixed target value or less, the control section 201 increases the aforesaid cleaning degree by controlling the pump 153. Meanwhile, when the value indicating the cleaning degree in the channel of the endoscope 100 exceeds the fixed target value, the control section 201 finishes a series of processing.

In step S19 of Fig. 4, the control section 201 determines whether or not the output of the pump 153 reaches the upper limit in terms of performance. When the control section 201 determines that the output of the pump 153 does not reach the upper limit in terms of performance, the control section 201 performs control to increase the output of the pump 153 in step S20 of Fig. 4, and thereafter, finishes a series of processing. Meanwhile, when the control section 201 determines that the output of the pump 153 reaches the upper limit in terms of performance, the control section 201 displays the warning to the effect that the target cleaning degree cannot be achieved (for example, "cannot achieve the target cleaning degree" or the like) on the display apparatus of the operation panel 25 in step S21 of Fig. 4, and thereafter, finishes a series of processing.

As described above, according to the endoscope automatic cleaning/disinfecting apparatus of the present embodiment, the cleaning power for the inside of the channel of the endoscope can be indirectly evaluated. As a result, according to the endoscope automatic cleaning/disinfecting apparatus of the present embodiment, the inside of the channel of the endoscope can be cleaned while high cleaning power is kept.

Further, as described above, according to the endoscope automatic cleaning/disinfecting apparatus of the present embodiment, the cleaning power for the inside of the channel of the endoscope can be evaluated based on the measurement value of the liquid flowmeter. More specifically, according to the endoscope automatic cleaning/disinfecting apparatus of the present embodiment, both the gas flowmeter and the liquid flowmeter do not have to be provided for the use of evaluating the cleaning power for the inside of the channel of the endoscope. Therefore, the conduit structure can be simplified, and the manufacturing cost can be reduced.

(Embodiment 3)

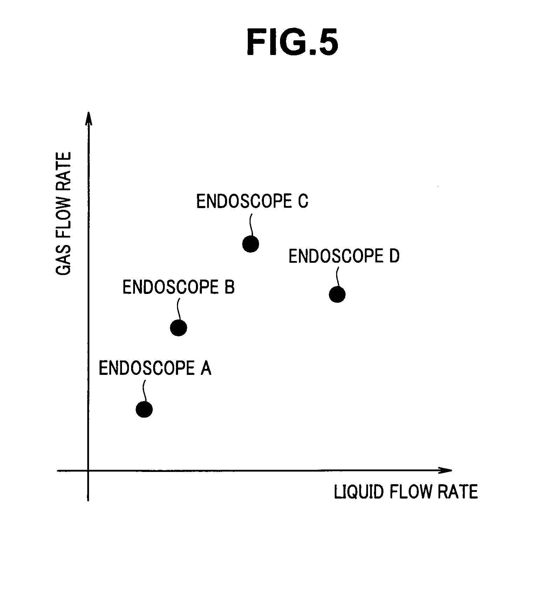

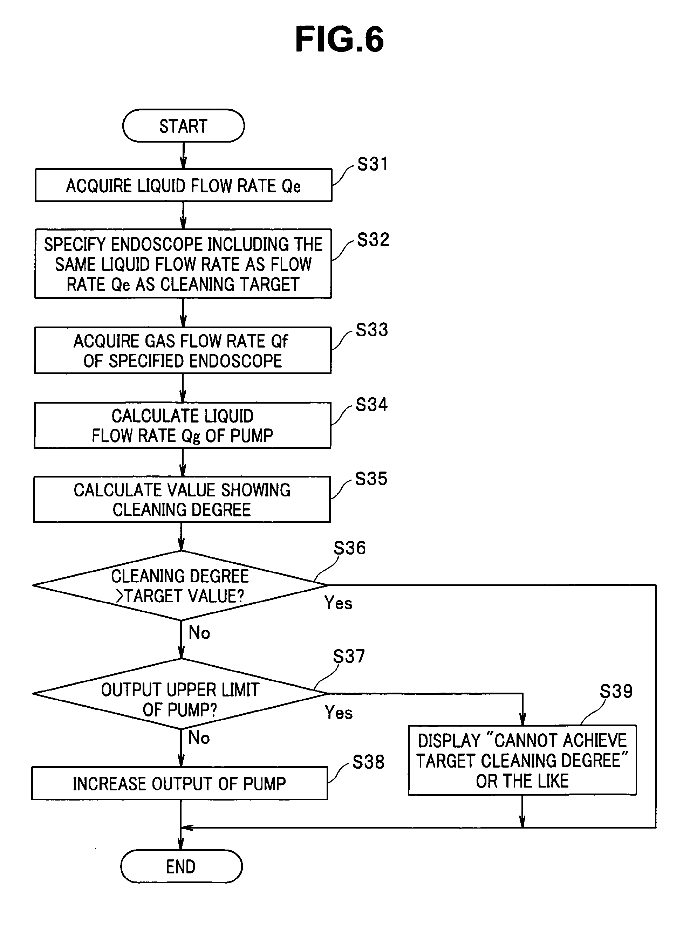

Figs. 5 and 6 relate to embodiment 3 of the present invention. In the present embodiment, the case will be described, in which the measuring apparatus 150 is configured as a liquid flowmeter which measures the flow rate of a liquid. In the following description, the detailed description of the components having the configurations similar to those in embodiments 1 and 2 will be omitted.

In the storage section 202 of the present embodiment, the flow rate data as the data of one-to-one correspondence of the liquid flow rate measured by actually passing a liquid into the channel of an endoscope and a gas flow rate measured by actually passing a gas into the channel of the aforesaid endoscope is written in advance for each of the endoscopes including the channel diameters and the conduit structures differing from one another. In concrete, the aforesaid flow rate data is written in the storage section 202, for example, in the scatter diagram of Fig. 5, or in the format analogous to the aforesaid scatter diagram. (The scatter diagram of Fig. 5 shows the case of four flow rate data being written in the storage section 202 as an example.) Further, in the storage section 202 of the present embodiment, the optimal mixture ratio of the gas and the liquid in the supply fluid supplied to the endoscope is written in advance.

Meanwhile, in step S31 of Fig. 6, the control section 201 of the control unit 200 acquires the measurement value of the flow rate of the liquid flowing in the fluid supply conduit 151 from the measuring apparatus 150. In step S31 of Fig. 6, the control section 201 regards the measurement value acquired from the measuring apparatus 150 as a flow rate Qe of the liquid flowing into the channel of the endoscope 100, and reads the aforementioned flow rate data from the storage section 202.

Next, in step S32 of Fig. 6, the control section 201 specifies the endoscope including the same liquid flow rate as the aforesaid liquid flow rate Qe as the cleaning target by checking the flow rate Qe of the liquid flowing in the channel of the endoscope 100 and the aforementioned flow rate data.

In step S33 of Fig. 6, the control section 201 acquires a gas flow rate Qf of the endoscope specified by the aforementioned check.

After the control section 201 reads the optimal mixture ratio of the gas and the liquid in the supply fluid to be supplied to the endoscope 100 from the storage section 202, the control section 201 calculates a liquid flow rate Qg of the pump 153 by dividing the gas flow rate Qf by the aforesaid mixture ratio in step S34 of Fig. 6.

Thereafter, in step S35 of Fig. 6, the control section 201 calculates the value indicating the cleaning degree in the channel of the endoscope 100 by obtaining the ratio of the liquid flow rate Qe and the liquid flow rate Qg, for example.

In step S36 of Fig. 6, the control section 201 determines whether or not the value indicating the cleaning degree in the channel of the endoscope 100 exceeds a fixed target value. When the value indicating the cleaning degree in the channel of the endoscope 100 is the fixed target value or less, the control section 201 increases the aforesaid cleaning degree by controlling the pump 153. Meanwhile, if the value indicating the cleaning degree in the channel of the endoscope 100 exceeds the fixed target value, the control section 201 finishes a series of processing.

In step S37 of Fig. 6, the control section 201 determines whether or not the output of the pump 153 reaches the upper limit in terms of performance. When the control section 201 determines that the output of the pump 153 does not reach the upper limit in terms of performance, the control section 201 performs control to increase the output of the pump 153 in step S38 of Fig. 6, and thereafter, finishes a series of processing. Meanwhile, if the control section 201 determines that the output of the pump 153 reaches the upper limit in terms of performance, in step S39 of Fig. 6, the control section 201 displays the warning to the effect that the target cleaning degree cannot be achieved (for example, "cannot achieve the target cleaning degree" or the like) on the display apparatus of the operation panel 25, and thereafter, finishes a series of processing.

As described above, according to the endoscope automatic cleaning/disinfecting apparatus of the present embodiment, the cleaning power for the inside of the channel of the endoscope can be indirectly evaluated. As a result, according to the endoscope automatic cleaning/disinfecting apparatus of the present embodiment, the inside of the channel of the endoscope can be cleaned while high cleaning power is kept.

Further, as described above, according to the endoscope automatic cleaning/disinfecting apparatus of the present embodiment, the cleaning power for the inside of the channel of the endoscope can be evaluated based on the measurement value of the liquid flowmeter. More specifically, according to the endoscope automatic cleaning/disinfecting apparatus of the present embodiment, both the gas flowmeter and the liquid flowmeter do not have to be provided for the use of evaluating the cleaning power for the inside of the channel of the endoscope. Therefore, the conduit structure can be simplified, and the manufacture cost can be reduced.

The present invention is not limited to the aforementioned embodiments, and various modifications and applications can be made within the range without departing from the gist of the present invention.

The present application claims benefit of Japanese Patent Application No. 2008-191225 filed in July 24, 2008 the contents of which are cited in the description, claims and the drawings of the present application.

**EP 2 305 098 A1**

**Claims**

1. An endoscope automatic cleaning/disinfecting apparatus which cleans and disinfects an endoscope, comprising:

   fluid supply means for supplying a fluid to an endoscope conduit disposed inside the endoscope;
   a fluid supply conduit which is connected to the fluid supply means, and supplies the fluid to the endoscope conduit;
   a connection connector which is provided at a distal end at a side of the endoscope, of the fluid supply conduit, and is for connecting the fluid supply conduit to the endoscope;
   fluid measuring means which is disposed beside the connection connector, and measures a state of the fluid in the fluid supply conduit; and
   control means which calculates a cleaning evaluation value of the endoscope conduit based on a measurement value obtained by the fluid measuring means, and controls the fluid supply means in accordance with a result of the calculation.

2. The endoscope automatic cleaning/disinfecting apparatus according to claim 1,
   wherein the fluid measuring means is a pressure sensor which converts a pressure inside the fluid supply conduit, or a stress received from the fluid into a voltage.

3. The endoscope automatic cleaning/disinfecting apparatus according to claim 1,
   wherein the fluid measuring means is a flow rate sensor which measures a flow rate of the fluid passing through a portion of the fluid measuring means in the fluid supply conduit as a state of the fluid.

4. The endoscope automatic cleaning/disinfecting apparatus according to claim 2 or 3,
   wherein the fluid measuring means measures a state of the fluid, the fluid being a gas, a liquid, or a mixture fluid with the gas and the liquid being mixed.

5. The endoscope automatic cleaning/disinfecting apparatus according to claim 1, further comprising:

   endoscope identifying means which identifies a type of the endoscope; and
   recording means which records a cleaning profile comprising at least one data of a pressure and a flow rate of a supply fluid to be supplied to the endoscope conduit, a mixture ratio of the supply fluid, and a supply timing of the supply fluid, which are set in accordance with the type of the endoscope.

6. The endoscope automatic cleaning/disinfecting apparatus according to claim 5,
   wherein the control means calculates the cleaning evaluation value corresponding to the type of the endoscope, based on identification information obtained from the endoscope identifying means, the cleaning profile recorded in the recording means, and the measurement value obtained by the fluid measuring means.

7. The endoscope automatic cleaning/disinfecting apparatus according to claim 5 or 6,
   wherein the control means controls the fluid supply means in accordance with the cleaning profile recorded in the recording means based on the identification information obtained from the endoscope identifying means.

8. The endoscope automatic cleaning/disinfecting apparatus according to any one of claims 1 to 7, further comprising warning means which executes warning when the control means determines that the measurement value measured by the fluid measuring means does not reach a predetermined target value.

9. The endoscope automatic cleaning/disinfecting apparatus according to claim 8,
   wherein the warning means is display means which displays various kinds of information.

10. The endoscope automatic cleaning/disinfecting apparatus according to claim 3,
    wherein the fluid measuring means is a liquid flowmeter which measures a flow rate of a liquid passing through the portion of the fluid measuring means in the fluid supply conduit.

11. The endoscope automatic cleaning/disinfecting apparatus according to claim 10,
    wherein the control means calculates a gas flow rate in the fluid based on a measurement value obtained by the fluid flowmeter, calculates the cleaning evaluation value of the endoscope conduit based on the calculation result of the gas flow rate, and controls the fluid supply means in accordance with the calculation result of the cleaning evaluation value. 12. The endoscope automatic cleaning/disinfecting apparatus according to claim 10, further comprising recording means in which flow rate data of one-to-one correspondence of a liquid flow rate measured by passing a fluid into the endoscope conduit and a gas flow rate measured by passing a gas into the endoscope conduit for each endoscope is written in advance,

wherein the control means specifies the endoscope to be a cleaning target based on the measurement value obtained by the liquid flowmeter and the flow rate data, calculates the cleaning evaluation value of the endoscope conduit based on the specified result, and controls the fluid supply means in accordance with a calculation result of the cleaning evaluation value.

# FIG.1

# FIG.2

DISPLAY APPARATUS — 25

SWITCH

— 200

— 201    — 202

CONTROL SECTION ↔ STORAGE SECTION

— 26

RADIO ID TRANSMISSION /RECEPTION UNIT

2

23  24

4

32

150

151

P 152

P 153

155

LIQUID/GAS MIXING SECTION

156

CP 154

AIR FLOW INLET PORT

EP 2 305 098 A1

# FIG.3

```
                    ( START )
                        │
                        ▼
        ┌───────────────────────────┐
        │      MEASURE STRESS        │──── S1
        └───────────────────────────┘
                        │
                        ▼
        ┌───────────────────────────┐
        │        CALCULATE           │──── S2
        │    CLEANING DEGREE         │
        └───────────────────────────┘
                        │
                        ▼         S3
                  ◇─────────────◇        Yes
                 ╱ CLEANING DEGREE ╲───────────────────────────┐
                 ╲ >TARGET VALUE?  ╱                            │
                  ◇─────────────◇                              │
                        │ No                                    │
                        ▼         S4                            │
                  ◇─────────────◇        Yes                    │
                 ╱ PUMP/COMPRESSOR ╲──────────────┐            │
                 ╲  LIMIT VALUE?   ╱               │            │
                  ◇─────────────◇                 │            │
                        │ No                       │            │
                        ▼         S5               ▼    S6      │
        ┌───────────────────────────┐   ┌───────────────────────┐
        │  INCREASE OUTPUT OF        │   │  DISPLAY "CANNOT       │
        │  PUMP/COMPRESSOR           │   │  ACHIEVE TARGET        │
        └───────────────────────────┘   │  CLEANING DEGREE"      │
                        │                │  OR THE LIKE           │
                        │                └───────────────────────┘
                        │◄───────────────────────┘            │
                        │◄─────────────────────────────────────┘
                        ▼
                    (  END  )
```

# FIG.4

START

ACQUIRE LIQUID FLOW RATE Qw — S11

CALCULATE OPERATION PRESSURE Pw OF PUMP CORRESPONDING TO LIQUID FLOW RATE Qw — S12

CALCULATE INNER DIAMETER SHAPE COEFFICIENT C — S13

ACQUIRE FUNCTION SHOWING CORRELATION OF PRESSURE LOSS $\Delta P$ AND GAS FLOW RATE Qa — S14

ACQUIRE PRESSURE LOSS $\Delta Pc$ AND GAS FLOW RATE Qc IN OPERATION POINT OF COMPRESSOR — S15

CALCULATE LIQUID FLOW RATE Qd OF PUMP — S16

CALCULATE VALUE SHOWING CLEANING DEGREE — S17

CLEANING DEGREE >TARGET VALUE? — S18
Yes

No

OUTPUT UPPER LIMIT OF PUMP? — S19
Yes

No

INCREASE OUTPUT OF PUMP — S20

DISPLAY "CANNOT ACHIEVE TARGET CLEANING DEGREE" OR THE LIKE — S21

END

14

# FIG.5

# FIG.6

```
                        ┌─────────────┐
                        │    START    │
                        └──────┬──────┘
                               │
              ┌────────────────▼────────────────┐  S31
              │   ACQUIRE LIQUID FLOW RATE Qe    │
              └────────────────┬────────────────┘
                               │
       ┌───────────────────────▼───────────────────────┐  S32
       │ SPECIFY ENDOSCOPE INCLUDING THE                │
       │ SAME LIQUID FLOW RATE AS FLOW                  │
       │ RATE Qe AS CLEANING TARGET                     │
       └───────────────────────┬───────────────────────┘
                               │
              ┌────────────────▼────────────────┐  S33
              │   ACQUIRE GAS FLOW RATE Qf       │
              │   OF SPECIFIED ENDOSCOPE         │
              └────────────────┬────────────────┘
                               │
              ┌────────────────▼────────────────┐  S34
              │   CALCULATE LIQUID               │
              │   FLOW RATE Qg OF PUMP           │
              └────────────────┬────────────────┘
                               │
              ┌────────────────▼────────────────┐  S35
              │   CALCULATE VALUE SHOWING        │
              │   CLEANING DEGREE                │
              └────────────────┬────────────────┘
                               │
                         S36   ▼
                    ◇ CLEANING DEGREE ◇ ─── Yes ──────────┐
                    ◇ >TARGET VALUE?  ◇                    │
                               │ No                        │
                         S37   ▼                           │
                    ◇ OUTPUT UPPER LIMIT ◇ ─── Yes ──┐     │
                    ◇ OF PUMP?          ◇             │  S39│
                               │ No                  ▼     │
              ┌────────────────▼────────────────┐  ┌───────────────────┐
              │   INCREASE OUTPUT OF PUMP    S38 │  │ DISPLAY "CANNOT   │
              └────────────────┬────────────────┘  │ ACHIEVE           │
                               │                    │ TARGET CLEANING   │
                               │                    │ DEGREE"           │
                               │                    │ OR THE LIKE       │
                               │                    └─────────┬─────────┘
                               │◄─────────────────────────────┘
                        ┌──────▼──────┐
                        │     END     │
                        └─────────────┘
```

## INTERNATIONAL SEARCH REPORT

| International application No. |
| --- |
| PCT/JP2009/061717 |

A.  CLASSIFICATION OF SUBJECT MATTER
*A61B1/12(2006.01)i*

According to International Patent Classification (IPC) or to both national classification and IPC

B.  FIELDS SEARCHED

Minimum documentation searched (classification system followed by classification symbols)
A61B1/12

Documentation searched other than minimum documentation to the extent that such documents are included in the fields searched
      Jitsuyo Shinan Koho          1922-1996   Jitsuyo Shinan Toroku Koho   1996-2009
      Kokai Jitsuyo Shinan Koho    1971-2009   Toroku Jitsuyo Shinan Koho   1994-2009

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| A | WO 2004/49925 A1  (Olympus Corp.), 17 June, 2004 (17.06.04), Page 25, line 20 & US 2005/65405 A1      & EP 1502538 A1 | 1-12 |
| A | JP 2004-202248 A  (Ethicon, Inc.), 22 July, 2004 (22.07.04), & US 2004/118437 A1      & EP 1433412 A1 | 1-12 |

☐  Further documents are listed in the continuation of Box C.          ☐  See patent family annex.

| | | |
| --- | --- | --- |
| * | Special categories of cited documents: | |
| "A" | document defining the general state of the art which is not considered   to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T"  later document published after the international filing date or priority date and not in conflict with the application but cited to understand the principle or theory underlying the invention

"X"  document of particular relevance; the claimed invention cannot be considered novel or cannot be considered to involve an inventive step when the document is taken alone

"Y"  document of particular relevance; the claimed invention cannot be considered to involve an inventive step when the document is combined with one or more other such documents, such combination being obvious to a person skilled in the art

"&"  document member of the same patent family

| Date of the actual completion of the international search | Date of mailing of the international search report |
| --- | --- |
|       10 July, 2009 (10.07.09) |       21 July, 2009 (21.07.09) |

| Name and mailing address of the ISA/ | Authorized officer |
| --- | --- |
|       Japanese Patent Office | |
| Facsimile No. | Telephone No. |

Form PCT/ISA/210 (second sheet) (April 2007)

**EP 2 305 098 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006230709 A

- JP 2008191225 A

Page **18**